Europäisches Patentamt

(19) European Patent Office

Office européen des brevets

(11) Publication number: **0 326 583 B1**

(12) **EUROPEAN PATENT SPECIFICATION**

(45) Date of publication of patent specification:
**23.10.91 Bulletin 91/43**

(21) Application number: **88900432.1**

(22) Date of filing: **07.12.87**

(86) International application number:
**PCT/US87/03245**

(87) International publication number:
**WO 88/04557 30.06.88 Gazette 88/14**

(51) Int. Cl.⁵: **A61L 27/00**

(54) **IMPLANTABLE DEVICES HAVING HYDROPHOBIC COMPONENT.**

(30) Priority: **17.12.86 US 943511**

(43) Date of publication of application:
**09.08.89 Bulletin 89/32**

(45) Publication of the grant of the patent:
**23.10.91 Bulletin 91/43**

(84) Designated Contracting States:
**DE FR GB IT**

(56) References cited:
**EP-A- 0 139 576**
**EP-A- 0 144 534**
**EP-A- 0 160 483**
**EP-A- 0 226 061**
**US-A- 3 833 002**
**US-A- 4 534 349**

(73) Proprietor: **ALLIED-SIGNAL INC. (a Delaware
corporation)
Columbia Road and Park Avenue P.O. Box
2245R
Morristown New Jersey 07960 (US)**

(72) Inventor: **TANG, Reginald Ting-Hong
5 Deerwood Trail
Warren, NJ 07060 (US)**
Inventor: **LARGMAN, Theodore
7 Upper Field Road
Morristown, NJ 07960 (US)**
Inventor: **MARES, Frank
32 Valley Forge Drive
Whippany, NJ 07981 (US)**
Inventor: **CHIU, Tin-Ho
754 Ridgewood Road
Millburn, NJ 07041 (US)**

(74) Representative: **Brock, Peter William et al
URQUHART-DYKES & LORD 91 Wimpole
Street
London W1M 8AH (GB)**

## Description

This invention relates to polymer-containing devices useful for implantation into living tissue for cellular regeneration. The devices are composed of at least one base component and at least one hydrophobic polymer component with a hydrophobicity that is equal to or greater than that of lactide.

The use of polymers and copolymers for implantation in living tissue has steadily increased over the last few decades. Medical applications of such polymers include absorbable sutures, intraosseous implants and slow-release drug delivery systems.

More recently, their use has been extended to microtubular tissue regeneration guidance channels, particularly the bioresorbable polymers. For example, bioresorbable materials have been used in the repair of injured nerves. Nerves with severed axons, but intact somas, may retain the capability of regrowing from the proximal stump to reconnect distally. Structures have been fabricated that serve as conduits for the regrowth and reconnection of severed nerves. After accomplishing their function, these guides gradually disappear from the host.

To be effective, these devices, commonly known as nerve channels, nerve guidance channels, nerve guidance tubes, nerve guides, or nerve tubes, must be made from materials that meet a wide range of biological and physicochemical prerequisites. The material must be bioresorbable, nontoxic, noncarcinogenic, nonantigenic, and must demonstrate favorable mechanical properties such as flexibility, suturability, and amenability to custom fabrication.

Further, it has been recently appreciated that these materials also must be capable of supporting cellular growth, to the extent that they actually exert a "neurotropic" effect. After exerting such an effect, they must also retain structural integrity to the extent necessary to maximize the number of axons reaching the distal stump to restore nerve function. This requires guidance channel biodegradation/resorption rates compatible with axon growth rates.

Heretofore a variety of materials have been proposed for use in the fabrication of prosthetic devices. For example, EP-A 160483 describes a tubular prosthesis consisting of a backing tube made of polyester such as Dacron and an inner lining having a non-polar hydrophobic surface of silicone or a fluorinated polymer. US-A 4534349 describes nerve guidance channels formed from a lactic acid polymer. EP-A 226061 describes prosthetic devices formed from polylactides and EP-A 144534 describes bioresorbable prosthetic devices formed from a polyester having krebs-cycle dicarboxylic acids at the chain ends.

## SUMMARY OF THE INVENTION

The present invention provides a prosthetic device suitable for implantation into living tissue for tissue regeneration and growth, said device comprising a base component which forms the framework of said device totally or partially coated with a bioresorbable polymeric coating component, said coating component having a hydrophobicity greater than that of the base component and greater than that of poly(lactide).

## DETAILED DESCRIPTION OF THE INVENTION

The devices of the present invention are suitable for implantation into living organisms to encourage cellular growth and regeneration of tissue. The devices comprise a base component that serves as the framework for the device and template for new cellular infiltration and the regenerative process. The devices also comprise a second component, a hydrophobic polymer with a hydrophobicity at least that of lactide as defined by the carbon-hydrogen to oxygen ratio.

The base substances of the invention can be any synthetic or natural homopolymer, copolymer, or any mixture thereof, that can serve as a framework for the desired device. Illustrative of such suitable polymeric substances are homopolymers, copolymers or mixtures of biodurable polymers such as silicone, silicone rubber, polyethylene, polyethylene terephthalate, polyfluoroethylene, polyphosphazene, polyurethane, segmented polyurethane, or the like. Also useful are biodurable metallic substances such as titanium, and alloys such as chromium-cobalt-molybdenum alloys, Titanium-Aluminum-Vanadium alloys, and the like.

In some cases, it may be desirable to employ, at least in part, a bioresorbable material to serve as the base component. Illustrative of such materials are those made from "Kreb's cycle acids" or materials capable of metabolism in biological systems through the Kreb's cycle. Such materials include the carboxylic acids, such as the alphahydroxy carboxylic acids and dicarboxylic acids; or copolymers or mixtures thereof. Illustrative of these are succinic acid, fumaric acid, oxaloacetic acid, L-malic acid, D-malic acid, glycolic acid, L-lactic acid, D-lactic acid, and any combination, including mixtures thereof. Particularly useful are the polyesters derived from dicarboxylic acids with butanediols, propanediols, pentanediols, hexanediols, and the like. Other examples include the dioxanones, β -hydroxy acid esters such as butyrate, valerate, and unsymmetrically and/or symmetrically substituted 1,4-dioxane-2,5-diones.

In some preferred embodiments, polylactides are used as the base component, especially poly(DL) lactide. As used herein, the term "polylactide" is equivalent to "poly (lactic acid)" as meaning a polymer of lactic acid. In particular, DL-lactide is a lactide derived from a roughly racemic mixture of lactic acid, and this nomenclature is interchangeable with (DL) lactic acid, and L-lactide is a lactide derived from L-lactic acid, this nomenclature being interchangeable with (L) lactic acid.

The above-mentioned substances may be readily obtained commercially or prepared by conventional polymerization processes, such as ring-opening polymerization, catalytic ester, ester interchange, and condensation reactions. Whether purchased or synthesized de novo, the chosen material should lend itself to formation of various shapes and to further lamination processes.

In the preferred embodiments of the present invention utilizing polylactide as the base polymer, the polymer may be prepared by polymerization of the monomeric lactide units using any acceptable process, such as ring opening polymerization, melt polymerization, and the like. Certain catalysts may be useful in such polymerization processes as the reaction can take place at a lower temperature. In particularly preferred embodiments, a catalyst such as stannous octoate is used, as it is postulated that the polymer molecular weight increases and molecular weight distribution decreases with its use. Other useful organometallic catalysts for such purposes include stannous caprylate, stannous diacylate, and stannic tetracylate. These types of catalysts are often preferred as it is believed that living systems can tolerate residual amounts of these components when implantable devices contain them.

A biocompatible plasticizer or plasticizers may be added to impart greater flexibility to the polymeric materials used in the present devices. Such plasticizers include, e.g., but are not limited to, acetyl tributyl citrate, acetyl triethyl citrate, tri-n-butyl citrate, triethyl citrate, and triacetin. In particular, bioresorbable triethyl citrate has been found to be useful.

The second component of the devices of the invention is a polymer that is "hydrophobic." As used herein, the term hydrophobic means lacking affinity for water, resulting in a tendency to diminish hydrolytic chain scission.

The hydrophobic component of the present invention is of a hydrophobicity that is capable of encouraging acceptance of the implanted device by surrounding tissues, and in particular encourges the influx of cells leading to neovascularization. This hydrophobicity is also such that in some cases, the polymer can be used as a layer to discourage water penetration. This results in the maintenance of structural integrity of the device when exposed to materials such as growth factors and the like, known to accelerate the processes of degradation. In this sense, the hydrophobic component has a tendency to diminish hydrolytic polymer chain scission.

The level of polymer hydrophobicity useful to serve these purposes is at least that of lactide and can be guaged by the ratio of carbon plus hydrogen:oxygen ratio which generally ranges from about 3.5:1 (lactide) to about 1000:1, or even greater such as in the case of polyolefin wax. Further, the hydrophobic component of the invention shall be substantially free of pendant hydroxy groups and hydroxy containing groups, including carboxylic acid functionalities. Illustrative of polymers useful as this second component are esters derived from any dicarboxylic acid with at least three carbons, such as succinic, malic, fumaric; diols, with at least two carbons such as ethane, propane, butane, pentane, hexane, heptane, octane, nonane, and their isomers; esters derived from any hydroxy carboxylic acid, excluding glycolic acid, such as any l-hydroxy carboxylic acid with carbon units of up to 10, from lactic acid to 2-hydroxy-decanoic acid; β-hydroxy carboxylic acid with carbon units of up to 10 from 3-hydroxypropionic acid, 3-hydroxybutanoic acid, to 3-hydroxydecanoic acid and their isomers; and substituted hydroxy acid from j-position to w-position and all their isomers, in particular, the hydroxyacids derived from caprolactone(s), valerolactone(s), butyrolactones, and the like; and any of the above monomers extruded with ether linkages such as 1,4-dioxan-2-one, variously alkyl-substituted 1,4-dioxane-2-ones and material commonly known as segmented polyether esters. Any copolymers composed of any of the above units is also within the contemplation of the above invention.

The base component and hydrophobic polymer are combined to form the devices of the invention. For example, one distinct portion of the device may comprise the base component, while another portion comprises the hydrophobic component.

The ratio of base component to hydrophobic polymer component varies according to intended use of the implantable device. The hydrophobic component may be of such a nature that it is able to impart some structural and mechanical integrity to the device in a given situation, and thus the amount of base component required to provide support and a framework for the device is relatively small. In other embodiments, as for example tubular conduits with an open lumen that is susceptible to collapsing, a relatively larger amount of base component may be required to provide the necessary support and tubular framework. In some cases, a harder, more crystalline material, such as l-lactide, glycolide, d- or l- β -hydroxybutyrate and the like might be suitable

3

in this regard.

In the preferred embodiments, each component is formed into a layer. The layering may take place during fabrication of the polymer components themselves or post forming. For example, coextrusion of the various layers can be effectuated, the layers then subjected to press or vaccuum molding or stretching into the desired shape forming a laminated structure. Examples of other standard lamination procedures include techniques utilizing solution casting, melt casting, dipping from solution, blow molding, cross-head extrusion or concentric extrusion.

In devices to be implanted in living tissue, it is preferred that hygenic and clean-room conditions for manufacturing of medical articles be followed. For example, when solution formation is utilized, polymer solutions are commonly filtered before use in a laminar-flow hood to insure that the devices are prepared under clean-room conditions.

The devices of the present invention are particularly suited to be used in conjunction with growth or "tropic factors", which are preferred to encourage the growth and survival of various classes of cells in tissue culture, these factors most often being macromolecular proteins. The hydrophobic component maintains the structural integrity of the implants by providing a barrier to the degradative effects of the growth factors which cause premature hydrolysis, cellular infiltration, and swelling. This is particularly notable in tubular conduits where the lumen of the tubes collapse. Of particular interest are neuronotrophic factors for use in layered implantable nerve conduits. Of these growth factors may be mentioned such substances as collagen, fibrinogen, fibronectin, and laminin.

These substances may be obtained in pure form, or mixed with each other or mixed with some neutral carrier such as gelatinous material and the like. Combinations of growth factors are also within the contemplation of the invention.

Using a tubular device with at least an internal layer of a hydrophobic polymer as an example, tropic factors may be added to the lumen of the tube itself in amounts sufficient to enhance the regeneration of tissue upon implantation. The tropic factor is added prior to implantation of the device, usually in sufficient amount to coat the inside of the tube itself, by any convenient technique, such as for example, injection filling that coats the inner surface or completely fills the lumen. Concentrations of approximately 0.01 mg/ml to 100 mg/ml, and particularly 0.1 mg/ml to 10 mg/ml have been found to be particularly useful.

The polymer components of the present invention may be fabricated into various devices, which can take many forms depending on intended use. Some contemplated forms include solid products such as orthopaedic pins, clamps, screws, or plates, clips, staples, vascular implants or supports and nerve channels or supports. Other medical devices could include fibrillar products, knitted, woven or felted such as velours, burn dressings, hernia patches, absorbant papers or swabs, medicated dressings, facial substitutes, gauze, fabric, sheet, felt or sponge for hemostasis, dental packs and breast prostheses. A good description of the formation of bioresorbable materials as matted surgical dressings may be found in U.S. Patent No. 3,937,223 to Roth. Other devices include slowly digestible ion-exchange resins and slow release devices in the form of pills or pellets.

## DETAILED DESCTIPTION OF THE PREFERRED EMBODIMENTS

Particularly useful devices according to the present invention are layered implantable tubes or conduits of varying shapes, lengths and diameters, particularly nerve guidance channels and the like.

In the preferred embodiments of the present invention, polylactide is fabricated for use as the base polymer in the nerve guidance devices using a melt polymerization procedure with stannous octoate as the catalyst. The requisite parts per million (ppm) of catalyst range from about 5 to about 800, a particularly preferred amount being about 75 ppm -200 ppm. Reaction time ranges from about 4 hours to about 168 hours, with 6 hours being preferred. Reaction temperatures range from about 75° to 240°C, with about 180°C being preferred.

Polymers are generally polydisperse or heterogeneous in molecular weight. To improve the physical properties of a polymer product in the preferred embodiments, it is thus desirable to control the molecular weight distribution by the use of fractionation procedures. The molecular weight distribution is commonly calculated as dispersity, which is the weight average molecular weight divided by the number average molecular weight to show how wide a range of molecules is included. Dispersity of a polymer for use in these nerve guidance channels is preferred to be less than about 10.0; more preferred is a dispersity number of less than about 3.0; most preferred is about 1.0-1.9.

To obtain polymers of different molecular weights, fractional precipitation of the lactide polymer can be achieved using a "good-solvent" such as chloroform or dioxane and a "non-solvent" such as water, methanol, or the like. Polymers of narrow molecular weight distribution are also obtainable in this manner. Fractional recrystallization may be carried out by selecting a solvent with a boiling point higher than the dissolving temperature of the polymer. Upon cooling slowly, the low molecular weight fraction stays in solution, and the higher molecular weight fraction precipitates. Polymers of different weight average molecular weights and distribution can be judiciously combined to obtain a material of desired weight average molecular weight and distribution for different purposes.

In the case of the nerve guidance channel, the particular configuration of such tubes may vary according to the size and shape of the nerve to be repaired, and whether the intended repair is to take place in human surgery or in surgery involving other animal species.

With respect to nerve guidance channels, U.S. Patent No. 3,833,002 to Palma discloses various sizes and shapes that may be employed. Lengths of the tubes, internal diameters, and tubular wall thicknesses may vary according to intended use. The length of the tube would ordinarily be commensurate with the size of the gap to be repaired, also allowing extra tubing in which to insert nerve stumps. Particularly useful lengths range from about 3mm to about 3 feet.

The number of layers of each polymer component varies according to intended use. Any number of layers are within the contemplation of the invention. In a particularly preferred embodiment, a nerve channel comprises lactide as the base polymer component and is dipped into a solution of poly β-hydroxybutyrate-covalerate, or polycaprolactone. Once the coating layer drys, an envelope of the hydrophobic ester forms around the tube. The internal diameters of tubes so produced commonly range from about 0.013mm to 5.00mm. The outside wall thicknesses generally range between about 0.08mm to 3.0mm. A preferred range is 0.10 to 1.0mm in outside wall thickness.

The devices of the present invention may also be sterilized by means of the techniques usually employed by surgery as long as extensive decomposition of the material does not result. For example, sterilization with ethylene oxide at room temperature may be employed.

The following examples illustrate certain preferred embodiments of the invention and are not definitive of scope.

## POLYMER PREPARATION EXAMPLE I

A catalyst solution containing 2.49mg/ml of stannous octoate dissolved in THF was prepared. Two mls of the stanneous octoate solution was added to 25g of DL-lactide to achieve 200 parts per million. This mixture was then heated under an inert atmosphere for 6 hours at 180°C. The molecular weight average of the resulting polymer was determined to be approximately 178,000 in the absence of solvent weight. Molecular weight was determined by gel permeation chromatography, calibrated against polystyrene standards in THF.

## POLYMER PREPARATION EXAMPLE II

Preparation of poly(DL,lactide) PDLA was effected as follows.

Seventy four grams of recrystallized DL lactide was charged to a Teflon reactor along with 74 microliters of 10% stannous octoate in toluene. The reactor was fitted with a nitrogen inlet, thermocouple and an anchor stirrer. The vessel contents were heated by means of an oil bath. A Servodyne gauge and chart recorder were used to monitor the viscosity of the polymer melt.

After stirring for 70 minutes under a nitrogen blanket, the viscosity rose rapidly. The oil bath temperature was held at 190-200°C for five hours while the internal thermocouple registered 155°C.

A 30g aliquot of the polymer was dissolved in acetone and then precipitated with water in a Waring blender. The recovered solids were washed thoroughly with methanol and further granulated in a Waring blender. Finally, the solids were dried in a vacuum oven for 2 days at room temperature, and 24g of polymer were recovered. Reduced viscosity of the polymer was $\eta_{sp/c} = 2.10$, (0.1% in dioxane).

The molecular weight average was determined by gel permeation chromatography to be approximately 207,000 in the absence of solvent weight.

Polymers of various molecular weights and distribution were obtained by fractional precipitation.

## POLYMER PREPARATION EXAMPLE III

A catalyst solution containing 2.22 mg/ml of stannous octoate in tetrahydrofuran was prepared. 0.72 ml of the aforemention stannous octoate solution was added to 9.0 gram of recrystallized D-L-lactide to achieve 178 parts per million. This mixture was then sealed in a glass ampoule under vacuum and heated at 182°C in an oven for 6 hours. The weight average molecular weight of the resulting polymer after dissolving in acetate and reprecipitated with anhydrous methanol was determined to be 234,000. Molecular weight, and molecular weight distribution were determined by gel permeation chromatography in tetrahydrofuran which was calibrated with polystyrene standards at room temperature.

## Implantation Device Preparation - Example I

Cleaned tungsten mendrels (J.75 mm diameter) were repeatedly dipped into a chloroform solution of poly β-hydroxylbutyrate-(ICI ref. MBL/100/58: BXIC 83/2) or copoly (80/20 β-hydroxylbutyrate/valerate)-[ICI ref. MBL/100/58, BXP/V/3 (EE)] until the outside diameters, OD, were between 0.81 to 0.85 mm. The mandrels were then dipped into a poly D,L-lactide (235k MW) plus two percent triethylcitrate in tetrahydrofuran until the OD's were 1.00 to 1.05 mm. After drying overnight, the mandrels were dipped into the β-hydroxyl polyester chloroform solution again until a final OD of 1.1 to 1.14 mm were reached. After drying overnight, the nerve channels were separated from the mandrels inside the laminar flow hood. To produce the end caps, the nerve channels were cut to the desired lengths, they were placed back onto the mandrels with approximately 2 to 3 mm of the tungsten wire extending from the end of the nerve channel. Each end was then dipped four times into a diluted coating solution. After drying, the ends were trimmed and examined under 70 x magnification to assure the middle layer was completly covered.

## Implantation Device Preparation Example II

Five 1.00 mm diameter cleaned tungsten mendrels were repeatedly dipped into a tetrahydrofuran solution of 234k poly D,L-lactide polymer until the O.D.'s were 1.3 mm. After drying overnight inside the laminar flow hood, the nerve channels were stripped from the mandrels. A 10% chloroform solution of poly ε-caprolactone from Union Carbide [polycaprolactone polymer PCL-300 Lot 22040 (M.W. = 10,000) or PCL-700 Lot #6578 (M.W. = 40,000)] was coated onto the inside lumen only of the 234k polylactide nerve channels individually inside the laminar flow hood. The final inside diameter average was 0.87 mm and the inner surface under 70 x magnification was like orange skin but coverage was complete.

Clean-room conditions were maintained during the preparation of the nerve channels.

Six 2mm diameter tungsten mandrels were cleaned by heating to read hot with the oxidizing propane torch flame. After cooling down, they were dipped twice into a 10% by weight 172,000 molecular weight of poly D,L-lactide with 2% triethyl citrate in the beginning and at the end. In between, the mandrels were dipped twice into a 5% by weight 229,000 molecular weight of poly L-lactide. The final outside diameter was 2.50 ± 0.10 mm. All dippings were done inside a Class 100 laminar flow hood and all polymer solutions were pre-filtered with a 0.2 micron filter.

Clean-room conditions were maintained during the preparation of the nerve channels.

## IMPLANTATION STUDIES I

### A. Mouse Sciatic Nerve Regeneration

Adult anesthetized C57BL/6J mouse with a sciatic nerve transected had both the proximal stump and distal stump secured by a single 10-0 nylon suture and inserted into a 5-6 mm length of a layered nerve guide tube with collagen added, to give a final gap length of 3-4 mm. Postoperatively, the sciatic nerve of the animal, appropriately perfused for tissue studies, was again exposed and retransected 3 mm distal to the nerve guide tube. Nerve guides with enclosed regenerated nerves were then dissected out, post-fixed in 2% osmium tetroxide and processed for plastic embedding (DER, Ted Pella Inc.). Just before embedding, the tissue was divided into several segments for sampling at multiple cross-section levels. For most implants, five levels were sampled by one micron sections. These levels were: proximal sciatic stump at 1 to 2 mm proximal to the implant; three levels- (proximal, central, distal) within the tube 1 through the original gap, and the distal stump 1 to 2 mm distal to the implant. Data obtained in the central section was used for comparison. The number of myelinated axons in these sections was determined with a computer-control system. Selected blocks were then resectioned for electron microscopy.

## RESULTS

Myelinated axon counts for rats with nerve channels having collagen added to lumen:

|  | | Axon Counts (8 weeks) |
|---|---|---|
| 1. | PDLA - (core) | 7933 ± 1415 |
|  | - poly e caprolactone coating | (N = 3) |
| 2. | PDLA - (core) | 6294 ± 886 |
|  | - Biopol coating | (N = 5) |

PDLA Controls (even without addition of collagen which normally accelerates degradation) collapsed.

The following are myelinated axon counts for <u>mice</u> with nerve channels.

| | | 2 weeks | 4 weeks | 8 weeks |
|---|---|---|---|---|
| 1. | PDLA (core) copoly (80/20 b hydroxyl-butyrate/valerate) coating | 0 | 83,1713 | |
| 2. | PDLA (core) poly b hydroxybutyrate coating | 0 | 593 | |
| 3. | PDLA (core) poly e caprolactone coating | 0 | 1,430±76 (N=5) | 1,284± 1,001 (N=4) |
| 4. | PDLA (control) | 0 | 340±102 (N=5) | 627±185 (N=5) |

<u>Preparation of Implants III</u>

Poly l-lactide tubing with 2.75 mm O.D. and roughly 2.5 mm I.D. obtained as samples from Hexcel Medical Corp., 11711 Dublin Blvd., Dublin, CA 94568 was coated with polyolefin wax (Parafilm obtained from American Can Co., Greenwich, CT 06830). A hot dilute solution of the wax was prepared using toluene as solvent. To coat the inside surface of the tube, the hot wax solution was simply sucked into the tubing and released immediately. Air drying, emoved the residual toluene was removed. No peeling of the white film on the clear tubing was observed upon cutting or scraping with sharp objects. The outside surface of the tube was also similarly coated by immersion of the tube into the wax solution and retrieval of the thus-immersed tube.

<u>Preparation of Implants IV</u>

Porous titanium based (or so-called sintered titanium) implants such as those obtainable from Johnson and Johnson Products, Inc., Braintree, MA and porous Co-Cr-Mo alloy implants obtainable from 3M Corp., St. Paul, MN can be coated with a solution of the hydrophobic polymer solution such as those described in the previous samples.

Similarly, porous implants of polymethyl methacrylate (PMMA) can be obtained similar to those described in P.J. van Mullem, J.M. Vaandrager, J.P.A. Nicolai, and J.R. de Wijn, <u>Transactions of The Society of Biomaterials, April 1985</u>, p. 126. The porous PMMA structure, after the water soluble Carboxymethyl Cellulosegel is leached, could be coated with the hydrophobic polymer or its solutions.

**Claims**

1. A prosthetic device suitable for implantation into living tissue for tissue regeneration and growth, said device comprising a base component which forms the framework of said device totally or partially coated with a bioresorbable polymeric coating component, said coating component having a hydrophobicity greater than that of the base component and greater than that of poly(lactide).

2. The device of claim 1 wherein said coating component is a polyolefing wax.

3. The device of claim 1 wherein said coating component is a synthetic homopolymer or copolymer having a hydrophobicity of from greater than 3.5:1 to 1000:1.

4. The device of claim 1 wherein said base component is selected from the group consisting of biodurable polymers, metals and bioresorbable polymers.

5. The device of claim 4 wherein said base component is selected from the group consisting of bioresorbable polymers.

6. The device of claim 5 wherein said bioresorbable polymers are selected from the group consisting of polylactides, polyglycolides, and polymers derived from polymerization of Kreb cycle dicarboxylic acids and diols having at least 2 carbon atoms.

7. The device of claim 6 wherein said base component is a poly(lactide).

8. The device of claim 6 wherein said hydrophobic component is selected from the group consisting of poly (beta-hydroxybutyrate-co-valerate) and polycaprolactone.

9. The device of claim 8 wherein said hydrophobic component is polycaprolactone.

10. The device of claim 1 which is a nerve guidance channel.

11. The device of claim 7 wherein said base component is a polylactide and said hydrophobic component is selected from the group consisting of poly (beta-hydroxy butyrate-co-valerate) and polycaprolactone.

12. The device of claim 1 wherein the hydrophobic component is a coating layer around said base component.

## Patentansprüche

1. Zur Implantation in lebendes Gewebe zur Geweberegeneration und -wachstum geeignete prothetische Vorrichtung, wobei die Vorrichtung eine das Gerüst der Vorrichtung bildende Grundkomponente enthält, welche vollständig oder teilweise mit einer bioresorbierbaren polymeren Beschichtungskomponente beschichtet ist, wobei die Beschichtungskomponente eine größere Hydrophobizität als die Grundkomponente und als die von Polylactid aufweist.

2. Vorrichtung nach Anspruch 1, worin die Beschichtungskomponente ein Polyolefinwachs ist.

3. Vorrichtung nach Anspruch 1, worin die Beschichtungskomponente ein synthetisches Homopolymer oder Copolymer mit einer Hydrophobizität von mehr als 3,5:1 bis 1000:1 ist.

4. Vorrichtung nach Anspruch 1, worin die Grundkomponente aus der Gruppe biodurabler Polymere, Metalle und bioresorbierbarer Polymere ausgewählt ist.

5. Vorrichtung nach Anspruch 4, worin die Grundkomponente aus den bioresorbierbaren Polymeren ausgewählt ist.

6. Vorrichtung nach Anspruch 5, worin das bioresorbierbare Polymer aus Polylactiden, Polyglykoliden und Polymeren, welche sich aus der Polymerisation von Dicarbonsäuren und Diolen des Zitronensäurezyklus mit wenigstens 2 Kohlenstoffatomen ableiten, ausgewählt ist.

7. Vorrichtung nach Anspruch 6, worin die Grundkomponente ein Polylactid ist.

8. Vorrichtung nach Anspruch 6, worin die hydrophobe Komponente aus Poly-beta-hydroxybutyrat-covalerat und Polycaprolacton ausgewählt ist.

9. Vorrichtung nach Anspruch 8, worin die hydrophobe Komponente Polycaprolacton ist.

10. Vorrichtung nach Anspruch 1, welche ein Nervenleitungskanal ist.

11. Vorrichtung nach Anspruch 7, worin die Grundkomponente ein Polylactid und die hydrophobe Komponente aus Poly-beta-hydroxybutyrat-covalerat und Polycaprolacton ausgewählt ist.

12. Vorrichtung nach Anspruch 1, worin die hydrophobe Komponente eine Überzugsschicht um die Grundkomponente ist.

## Revendications

1. Dispositif prosthétique convenant pour l'implantation dans un tissu vivant pour la régénération et la croissance des tissus, ce dispositif comprenant un constituant de base formant la charpente de ce dispositif totalement ou partiellement revêtu d'un constituant de revêtement polymère bio-résorbable, ce constituant de revêtement ayant une hydrophobie supérieure à celle du constituant de base et supérieure à celle du poly(lactide).

2. Dispositif selon la revendication 1, dans lequel ce constituant de revêtement est une cire de polyoléfine.

3. Dispositif selon la revendication 1, dans lequel ce constituant de revêtement est un homopolymère ou copolymère synthétique ayant une hydrophobie supérieure à 3,5 : 1 à 1000 : 1.

4. Dispositif selon la revendication 1, dans lequel ce constituant de base est choisi parmi des polymères bio-résistants, des métaux et des polymères bio-résorbables.

5. Dispositif selon la revendication 4, dans lequel ce constituant de base est choisi parmi des polymères bio-résorbables.

6. Dispositif selon la revendication 5, dans lequel ces polymères bio-résorbables sont choisis parmi les polylactides, les polyglycolides, et les polymères provenant de la polymérisation d'acides dicarboxyliques du cycle de Krebs et de diols ayant au moins 2 atomes de carbone.

7. Dispositif selon la revendication 6, dans lequel ce constituant de base est un poly(lactide).

8. Dispositif selon la revendication 6, dans lequel ce constituant hydrophobe est choisi parmi le (béta-hydroxybutyrate-co-valérate) et la polycaprolactone.

9. Dispositif selon la revendication 8, dans lequel ce constituant hydrophobe est la polycaprolactone.

10. Dispositif selon la revendication 1, qui est un canal de guidage des nerfs.

11. Dispositif selon la revendication 7, dans lequel ce constituant de base est un polylactide et ce constituant hydrophobe est choisi parmi le poly(béta-hydroxy butyrate-co-valérate) et la polycaprolactone.

12. Dispositif selon la revendication 1, dans lequel le constituant hydrophobe est une couche de revêtement autour de ce constituant de base.